## Europäisches Patentamt

## European Patent Office

(11) Veröffentlichungsnummer: **0 012 945**
**B1**

## Office européen des brevets

(19)

(12) **EUROPÄISCHE PATENTSCHRIFT**

(45) Veröffentlichungstag der Patentschrift:
**29.12.82**

(51) Int. Cl.³: **G 01 N 27/56**

(21) Anmeldenummer: **79105149.3**

(22) Anmeldetag: **13.12.79**

(54) Vorrichtung zur Messung der Emission von gasförmigen, anorganischen Fluor- oder Chlorverbindungen.

(30) Priorität: **28.12.78 DE 2856490**

(43) Veröffentlichungstag der Anmeldung:
**09.07.80 Patentblatt 80/14**

(45) Bekanntmachung des Hinweises auf die Patenterteilung:
**29.12.82 Patentblatt 82/52**

(84) Benannte Vertragsstaaten:
**AT BE CH DE FR GB IT NL SE**

(56) Entgegenhaltungen:
**DE-A-2 455 602**
**FR-A-1 251 609**
**FR-A-1 391 158**
**FR-A-2 207 603**
**FR-A-2 392 383**
**US-A-3 001 917**
**TECHNISCHES MESSEN ATM, Jahrgang 45, Nr. 10, 1978, München, DE, U. FRITZE: »Ein schnell ansprechender kontinuierlicher Gasspurenmonitor mit ionensensitiver Elektrode«, Seiten 305—307**

(73) Patentinhaber: **SIEMENS AKTIENGESELLSCHAFT**
**Berlin und München, Postfach 22 02 61,**
**D-8000 München 22 (DE)**

(72) Erfinder: **Scheubeck, Egmont, Dresdner Strasse 14b,**
**D-8520 Erlangen (DE)**
Erfinder: **Boehner, Marianne, Löhestrasse 45,**
**D-8520 Erlangen (DE)**
Erfinder: **Blazevic, Gertrud, Dr., Daimlerstrasse 33,**
**D-8520 Erlangen (DE)**

Vorrichtung zur Messung der Emission von gasförmigen, anorganischen Fluor- oder Chlorverbindungen

Die Erfindung betrifft eine Vorrichtung zur Messung der Emission von gasförmigen, anorganischen Fluor- oder Chlorverbindungen, wobei in der Vorrichtung das zu untersuchende Abgas über eine beheizte Probenahmesonde mittels Pumpen in eine Absorptionsflüssigkeit und einen Flüssigkeitsabscheider gesaugt wird und wobei die Vorrichtung eine fluorionensensitive oder chlorionensensitive Elektrodenmeßkette zur potentiometrischen Erfassung der Fluor- oder Chlorionenkonzentration aufweist.

Die bisher eingesetzten Vorrichtungen zur Erfassung von gasförmigen, anorganischen Fluor- oder Chlorverbindungen, beispielsweise bei Müllverbrennungsanlagen, arbeiten mit diskontinuierlicher photometrischer oder potentiometrischer Meßwerterfassung (ANALYTICAL CHEMISTRY, Vol. 40, No. 11, 1968, S. 1658 – 1661; TÜ 13 (1972), Nr. 2 Febr., S. 42 – 45; Sonderdruck aus Ziegelindustrie, H. 10/1973, 26. Jahrg., »Kontinuierliche Messung von Fluoremissionen« v. G. Kassebeer; Fluoride Vol. 4, Nr. 1, Jan. 1971, S. 49 – 55). Die kürzeste Meßwertangabe erfolgt nur alle 5 Minuten, im allgemeinen alle 20 – 30 Minuten. Die bekannten Vorrichtungen genügen nicht den Anforderungen beim Einsatz in Industrieanlagen, wo automatisch sichere und genaue Analysenergebnisse erforderlich sind und die Erfassung der Schadstoffe und die Ausgabe der Meßwerte möglichst kontinuierlich erfolgen soll. Auch ist die Probenahmesonde starr und auf die spezielle Anlage abgestimmt, bei der sie eingesetzt ist und es ist insbesondere für Fluorwasserstoff keine sichere verlustfreie Überführung zur Meßzelle möglich. Es sind auch relativ lange Einstellzeiten bis zum konstanten Endpotential erforderlich.

Der Erfindung liegt die Aufgabe zugrunde, eine Vorrichtung zu schaffen, welche nicht mit den Nachteilen der bekannten Einrichtungen behaftet ist. Dabei geht es insbesondere darum, mit konstruktiv einfachen und zuverlässig wirkenden Mitteln eine automatische und kontinuierliche Erfassung mit kontinuierlicher Meßwertausgabe über Schreiber, Drucker u. dgl. von emittierten anorganischen, gasförmigen Fluor- oder Chlorverbindungen zu gewährleisten.

Die Lösung dieser Aufgabe ist im Kennzeichen des Anspruchs 1 angegeben. In der Praxis ist das Gerät mit einer Einrichtung zur automatischen Selbsteichung mit Eichstandardlösung versehen. Die Probenahmesonde ist flexibel, beheizbar und mit einer chemisch beständigen Auskleidung, vorzugweise aus Gold oder Platin versehen. Gasprobenahme, Konzentrationserfassung und Meßwertausgabe erfolgen kontinuierlich. Für einen Zeitraum von ca. 7 Tagen ist ein wartungsfreier und ununterbrochener Betrieb des Gerätes möglich. Ansprechzeiten von etwa 35 Sekunden sind gut erreichbar.

Zur analytischen Erfassung der emittierten Mengen an gasförmigen, anorganischen Fluor- oder Chlorverbindungen wird das Abgas bzw. die Abgasprobe über eine beheizte, flexible Probenahmesonde mittels einer Gasdosierpumpe in ein geeignetes birnenförmiges Absorptionsgefäß enthaltend kontinuierlich zugeführte Absorptionsflüssigkeit gesaugt. Nach der Absorption der gasförmigen Fluor- oder Chlorverbindungen aus dem Abgas erfolgt eine Entgasung der Flüssigkeit in einem zylindrischen Flüssigkeitsabscheider. Die die Fluor- oder die Chlorionen enthaltende Absorptionslösung wird mit einer Flüssigkeitspumpe in eine zylindrische Durchflußmeßzelle mit geringem Volumen gesaugt, wo die potentiometrische Erfassung der Fluor- oder Chlorionenkonzentration mit Hilfe einer fluorionensensitiven oder chlorionensensitiven Elektrodenmeßkette erfolgt. Anschließend fließt die Lösung in einen Abwasserbehälter.

Die Erfindung wird anhand eines Ausführungsbeispiels und der ein Ausführungsbeispiel darstellenden Zeichnung näher erläutert.

Durch die Fritte 34 erfolgt beim Durchführen von Probegas und Absorptionslösung eine intensive Vermischung zur quantitativen Absorption der zu erfassenden Fluor- oder Chlorverbindungen in der Absorptionslösung. Die Zuführung des Probegases über 38 erfolgt seitlich in die Zuleitung 39 für die Absorptionslösung 7 oberhalb des Austritts durch eine Fritte; die Abführung seitlich unterhalb über einen trichterförmigen Auslaß. Zur Elektrodenbefestigung in der Durchflußmeßzelle 14 haben sich Stopfen 37 aus Teflon® mit Gewinde 41 und je einer kombinierten Abdichtung 42 als besonders günstig erwiesen.

Beim zylindrischen Flüssigkeitsabscheider 9 hat es sich bewährt, die probengashaltige Absorptionsflüssigkeit über ein durchgehendes gerades Einlaßrohr 35 bis nahe an den Flüssigkeitsauslaß 40 so zu führen, daß ein tropfenweiser Austritt möglich ist. Das entweichende Gas wird am seitlichen oberen Ende des Flüssigkeitsabscheiders über ein gebogenes Rohr 36 abgeführt.

Als Durchflußmeßzelle 14 hat sich ein zylindrisches Glasgefäß mit 2 mm innerem Durchmesser und zwei Gewindeansätzen 44 zur Aufnahme der Stopfen aus Teflon® für die Befestigung der Elektroden bewährt. Gemäß einer vorteilhaften Ausführungsform hat die Durchflußmeßzelle 14 zwei Gewindeansätze 44 und Stopfen 37 aus Teflon® mit Gewinde 41 und eine kombinierte Abdichtung 42 aus verschiedenen Kunststoffen zur Elektrodenbefestigung. Im einzelnen zeigt

Fig. 1 ein Schema der Vorrichtung,

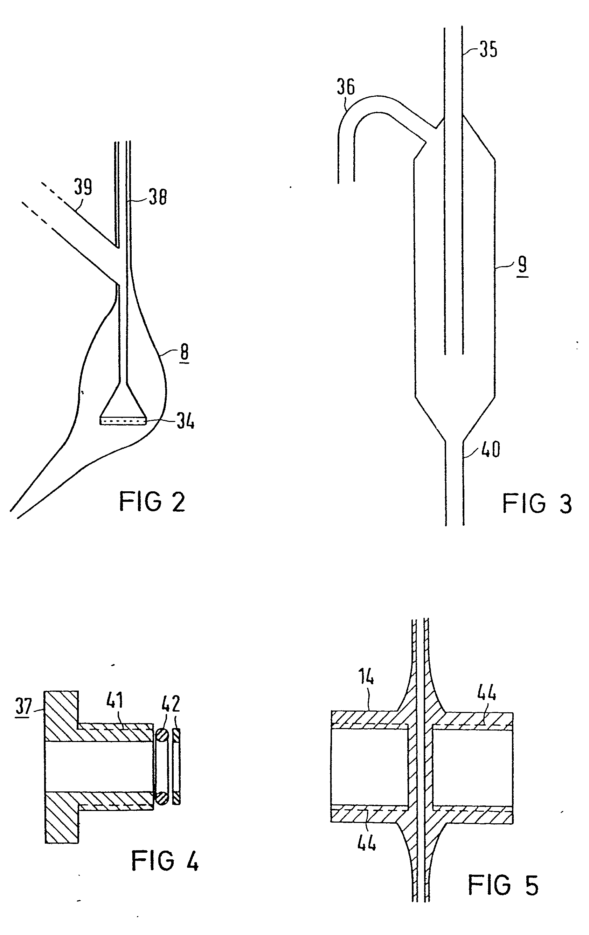

Fig. 2 ein Absorptionsgefäß im Schnitt nach Fig. 1,

Fig. 3 einen Flüssigkeitsabscheider im Schnitt nach Fig. 1,

Fig. 4 eine Elektrodenbefestigung mit Abdichtung nach Fig. 1,

Fig. 5 eine Meßzelle im Schnitt nach Fig. 1.

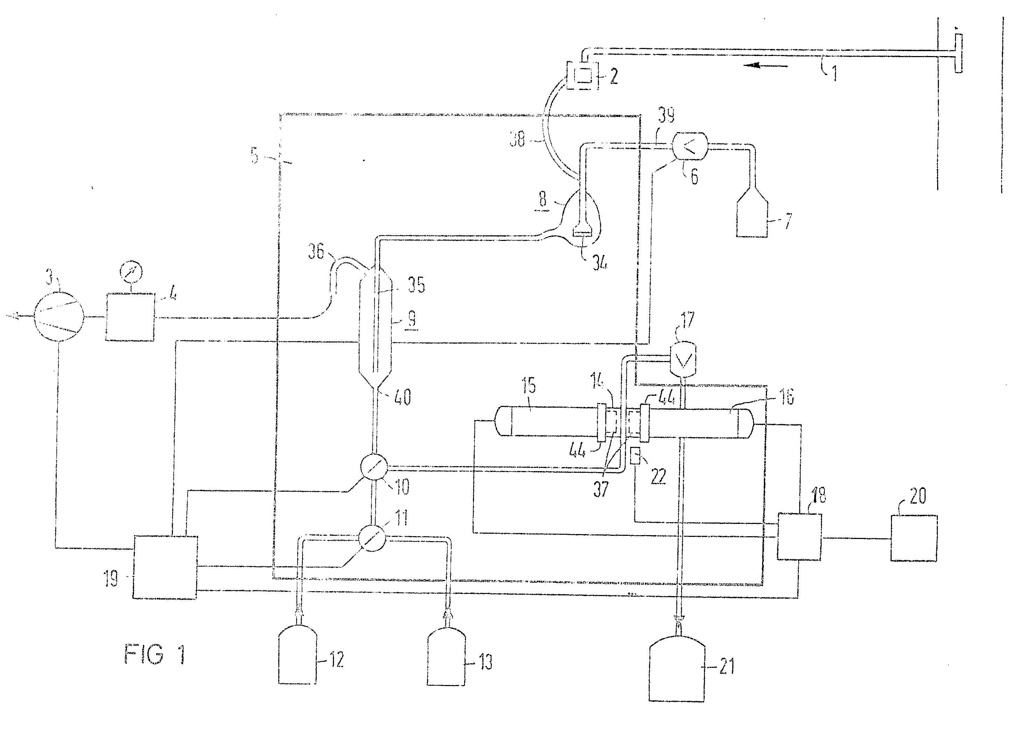

Die in Fig. 1 dargestellte Vorrichtung zeigt eine Gaspumpe 3. Durch diese und das Gasregelsystem 4 wird über eine beheizbare Probenahmesonde 1 und Filter 2 die Gasprobe über die seitliche Zuführung 38 durch das birnenförmige Absorptionsgefäß 8 gesaugt. Gleichzeitig wird Absorptionslösung 7 durch Zuleitung 39 mittels der Flüssigkeitspumpe 6 gefördert. Die nun fluoridhaltige Lösung wird mit der zweiten Flüssigkeitspumpe 17 aus dem zylindrischen Flüssigkeitsabscheider 9 durch die zylindrische Durchflußmeßzelle 14 mit der fluorionensensitiven Indikatorelektrode 15 und der Bezugselektrode 16 gesaugt. Das Absorptionsgefäß 8, der Flüssigkeitsabscheider 9 und die Durchflußmeßzelle 14 bilden mittels kurzer Schlauch- bzw. Rohrverbindungen eine Einheit und sind zusammen mit den Magnetventilen 10 und 11 auf einer Platte 5 befestigt. Das Elektrodenpotential wird linear über den Verstärker 18 verstärkt und auf dem Schreiber 20 registriert. Das Abwasser wird in den Behälter 21 geleitet. Ein Thermofühler 22 dient zur Kompensation des Temperatureinflusses auf den Meßwert. Die Eichung kann automatisch nach einem 52 Minuten dauernden Meßcyclus erfolgen, wobei die zeitliche Eichung wählbar ist. Dauer der Eichung 8 Minuten. Durch entsprechendes Umschalten der Magnetventile 10 und 11 wird zuerst Eichlösung I aus 12 und daraufhin Eichlösung II aus 13 durch die Meßzelle 14 gesaugt. Falls Abweichungen des Elektrodenpotentials vom Sollwert auftreten, wird der Verstärker 18 mit Steuerung 19 automatisch nachgeeicht.

Die Pumpe 3 für die Gasentnahme sowie die Pumpe 6 für die Absorptionslösung werden während des Eichcyclusses automatisch abgeschaltet.

In analoger Weise kann Chlorid aus gasförmigen, anorganischen Chlorverbindungen analytisch unter Einsatz einer chlorionensensitiven Indikatorelektrode erfaßt werden, wobei Gasprobenahme, Konzentrationserfassung und Meßwertausgabe kontinuierlich erfolgen.

### Patentansprüche

1. Vorrichtung zur Messung der Emission von gasförmigen, anorganischen Fluor- oder Chlorverbindungen, wobei in der Vorrichtung das zu untersuchende Abgas über eine beheizte, flexible Probenahmesonde mittels Pumpe in eine Absorptionsflüssigkeit und einen Flüssigkeitsabscheider gesaugt wird und wobei die Vorrichtung eine fluorionensensitive oder chlorionensensitive Elektrodenmeßkette zur potentiometrischen Erfassung der Fluorionenkonzentration oder Chlorionenkonzentration aufweist, dadurch gekennzeichnet, daß die Sonde (1) mit einem chemisch resistenten Werkstoff ausgekleidet ist, daß ein die angesaugten Abgase in der angesaugten Absorptionsflüssigkeit (7) aufnehmendes birnenförmiges Absorptionsgefäß (8) mit einer Fritte (34) sowie eine zylindrische Durchflußmeßzelle (14) vorgesehen ist, die mit dem Absorptionsgefäß (8) über den dazwischenliegenden eine zylindrische Form aufweisenden Flüssigkeitsabscheider (9) verbunden ist.

2. Vorrichtung nach Anspruch 1, dadurch gekennzeichnet, daß der zylindrische Flüssigkeitsabscheider (9) ein durchgehendes gerades Einleitungsrohr (35) bis kurz vor den Flüssigkeitsauslaß (40) und einen gebogenen Gasauslaß (36) enthält.

3. Vorrichtung nach Anspruch 1 und 2, dadurch gekennzeichnet, daß die Durchflußmeßzelle (14) zwei Gewindeansätze (44) und Stopfen (37) aus Polytetrafluoräthylen mit Gewinde (41) und eine kombinierte Abdichtung (42) zur Elektrodenbefestigung aufweist.

### Claims

1. Apparatus for measuring the emission of gaseous, inorganic fluorine or chlorine compounds, in which apparatus the waste gas to be investigated is sucked by means of a pump into an absorbent liquid and a liquid separator through a heated, flexible sampling probe, and in which the apparatus has an electrode measuring chain, which is sensitive to fluorine or chlorine ions, for the potentiometric determination of the fluorine ion or chlorine ion concentration, characterised in that the probe (1) is coated with a chemically-resistant material and that a pear-shaped absorption vessel (8) which absorbs the sucked-in waste gases in the sucked-in absorption liquid, is provided with a fritted element (34) and with a cylindrical through-flow measuring cell (14) which is connected to the absorption vessel (8) by way of the intermediately arranged liquid separator (9) which is of cylindrical shape.

2. Apparatus as claimed in claim 1, characterised in that the cylindrical liquid separator (9) contains a continuous straight inlet pipe (35) extending to shortly before the liquid outlet (40) and a curved gas outlet (36).

3. Apparatus as claimed in claim 1 and 2, characterised in that the through-flow cell (14) has two threaded attachments (44) and plugs (37), which are made of polytetrafluoroethylene having threads (41), and a compound seal (42) for electrode fixing purposes.

### Revendications

1. Dispositif de mesure du dégagement de composés minéraux gazeux fluorés ou chlorés, dans lequel dans le dispositif le gaz résiduaire à analyser est aspiré, à l'aide d'une pompe, par l'intermédiaire d'une sonde souple chauffée de prélèvement d'échantillons, dans un liquide d'absorption et dans un séparateur de liquide et dans lequel le dispositif présente une chaîne de mesure à électrodes sensibles aux ions fluor ou sensibles aux ions chlore pour la détection potentiométrique de la concentration d'ions fluor ou de la concentration d'ions chlore, caractérisé en ce que la sonde (1) est revêtue intérieurement d'un matériau résistant chimiquement, en ce qu'il est prévu un récipient (8) piriforme d'absorption, recevant le gaz résiduaire aspiré dans le liquide (7) d'absorption aspiré et ayant une fritte (34), ainsi qu'une cellule (14) cylindrique de mesure du débit, qui communique avec le récipient (8) d'absorption par le séparateur (9) de liquide interposé ayant une forme cylindrique.

2. Dispositif suivant la revendication 1, caractérisé en ce que le séparateur (9) de liquide contient un tube (35) d'entrée continu, rectiligne se terminant peu avant la sortie (40) pour le liquide et une sortie (36) coudée pour le gaz.

3. Dispositif suivant la revendication 1 ou 2, caractérisé en ce que la cellule (14) de mesure du débit présente deux raccords (44) taraudés et deux bouchons (37) en polytétrafluoroéthylène à filetage et une garniture (42) combinée d'étanchéité pour la fixation d'électrodes.

FIG 1

FIG 2

FIG 3

FIG 4

FIG 5

7